# EUROPÄISCHE PATENTSCHRIFT

(11) **EP 0 377 799 B1**
(45) Veröffentlichungstag und Bekanntmachung des Hinweises auf die Patenterteilung: **15.06.1994**
(21) Anmeldenummer: 89119994.5
(22) Anmeldetag: 27.10.1989
(51) Int. Cl.: A61L 2/14

(54) **Verfahren zum Sterilisieren oder Reinigen von Gegenständen**
Method for sterilizing or cleaning articles
Procédé de stérilisation ou de nettoyage d'objets

(30) Priorität: 13.01.1989 DE 3900883
(43) Veröffentlichungstag der Anmeldung: 18.07.1990
(73) Patentinhaber: TECHNICS PLASMA GMBH, 85551 Kirchheim (DE)
(72) Erfinder: Möhl, Wolfgang, Dr., D-8011 Kirchheim b. München (DE); Liebel, Gerhard, D-8011 Kirchheim b. München (DE)
(74) Vertreter: Boeters, Hans Dietrich, Dr.

(56) Entgegenhaltungen:
- WO-A-90/04418
- DE-A- 3 000 709
- FR-A- 1 571 833
- FR-A- 2 355 511

## Beschreibung

Es ist bereits bekannt, Gegenstände, wie Werkzeuge, Behälter oder deren Teile, die in der Medizin, der Biologie, der Lebensmitteltechnologie oder der Pharmazie Verwendung finden, mit Hilfe von Heißdampf, Ethylenoxid oder ionisierender Strahlung zu sterilisieren. Bei diesen bekannten Verfahren werden den zu sterilisierenden Gegenständen anhaftende Bakterien oder Keime abgetötet.

In der DE-A-2 260 854 wird eine Vorrichtung vorgeschlagen, die in einer Sterilisierkammer mit Hilfe eines Radiofrequenzfeldes ein Gasplasma erzeugt, mit dessen Hilfe sich eine Verringerung des Mikrobenbefalls bis zu 99 % erreichen lassen soll, jedoch keine vollständige Sterilisierung und Reinigung der behandelten Gegenstände.

Die Lehre der DE-A-3 000 709 hat es sich zur Aufgabe gemacht, für Sterilisationen die scharfen Anforderungen des AOAC-Abtötungstests zu erfüllen. Dazu wird vorgeschlagen, daß man Gegenstände mit Hilfe eines Niederdruckplasmas sterilisiert, das durch eine elektromagnetische Strahlung im Frequenzbereich von 1 bis 100 MHz oder 100 bis 300 000 MHz = 0,1 bis 300 GHz erzeugt wird, wobei das Niederdruckplasma zwingend mindestens 10 mg/l eines Aldehyds enthält. Es soll ein Sterilisationsgrad erreichbar sein, bei dem in keiner von 10 Proben ein Wachstum feststellbar ist.

Aus DE-A-3 000 709 sind Maßnahmen zum Sterilisieren von Gegenständen bei mikrobiologischer Verunreinigung mit Hilfe eines Niederdruckplasmas bekannt, wobei man das Niederdruckplasma durch eine elektromagnetische Strahlung von 2,45 GHz in einem aldehydfreien Medium erzeugt. Dieser Stand der Technik will die scharfen Anforderungen des AOAC-Spurenabtötungstests erfüllen und lehrt dazu, daß sich im Schnitt bei Gegenwart eines Aldehyds bessere Sterilisationen erzielen lassen. Zusätzlich ist aus WO-A-90/04418 ein Verfahren zum Sterilisieren von Gegenständen mit Hilfe eines Niederdruckplasmas einer EM-Strahlung von 2,45 GHz in einem aldehydfreien Medium bei einem Druck im Bereich von 1,3 bis 1,3 × 10⁻³ mbar (1000 bis 1 »mHg) bekannt. Dieser Stand der Technik ist auf eine Sporenabtötung bzw. Sterilisation gerichtet, wobei angegeben wird, daß die Zerstörung der DNA-Moleküle der Mikroorganismen auf die direkte Ionisation im Plasma zurückzuführen ist.

Demgegenüber ist es Aufgabe der Erfindung, eine vollständige Sterilisation mit vollständiger Entfernung von "Bakterienleichen" und auch Pyrogenen zu erreichen und dabei ohne Aldehyd auszukommen.

Diese Aufgabe wird erfindungsgemäß durch ein verfahren zum Sterilisieren und Reinigen von Gegenständen von mikrobiologischen Verunreinigungen mit Hilfe eines Niederdruckplasmas gelöst, bei dem man das Niederdruckplasma durch eine elektromagnetische Strahlung von etwa 2,45 GHz bei einem Druck im Bereich von 5 × 10¹ bis 0,5 × 10⁻⁴ mbar in einem aldehydfreien Medium erzeugt, wobei das Verfahren dadurch gekennzeichnet ist, daß man die eingesetzten Gegenstände bei der Sterilisation und Reinigung mit Hilfe eines IR-Strahlers bei erhöhter Temperatur hält und sterilisiert und reinigt.

Bei den erfindungsgemäßen Verfahren bewirken die aktiven Gasteilchen des Niederdruckplasmas, also beispielsweise Ionen oder Radikale, eine Oxidation aller organischen Materie zu gasförmigen und damit flüchtigen Verbindungen, beispielsweise Kohlenmonoxid, Kohlendioxid und Wasser. Dadurch werden Bakterien und Keime, die den zu behandelnden Gegenständen anhaften, nicht nur abgetötet, sondern bei ausreichender Einwirkungsdauer des Niederdruckplasmas auch völlig entfernt.

Die Wirkung des Plasmas wird durch UV-Strahlung unterstützt, die durch das Plasma erzeugt wird.

Als Medium des Niederdruckplasmas kann man beispielsweise Sauerstoff, Stickstoff, Tetrafluormethan, ein Edelgas oder deren Gemische verwenden, jedoch sind für den Fachmann auch andere Gase oder Gasmischungen denkbar.

Man kann beispielsweise in einem Druckbereich von 10¹ bis 10⁻³ mbar arbeiten.

Das Niederdruckplasma wird in bekannter Weise dadurch erzeugt, daß man durch Hochfrequenzeinkoppelung das gewählte Medium einer elektromagnetischen Strahlung aussetzt, und zwar einer Strahlung von etwa 2,45 GHz. Eine Vorrichtung, die in diesem Mikrowellenbereich betrieben werden kann, ist beispielsweise in der deutschen Patentanmeldung (dem deutschen Patent) P 37 38 352.3 beschrieben.

Die in die erfindungsgemäßen Verfahren einzusetzenden Gegenstände können dem Niederdruckplasma in dem Reaktor ausgesetzt werden, in dem das Niederdruckplasma erzeugt wird. Sie können aber auch in einer separaten mit dem Reaktor verbundenen Kammer dem Niederdruckplasma ausgesetzt werden.

Für große Kammern mit einem Volumen von mehr als etwa 20 Litern wird die Hornstrahleranordnung oder die ECR-Plasma-Stromquelle zur Plasmaerzeugung bevorzugt, um das Plasma großvolumig zu verteilen und möglichst wirksam anzuregen. Für Vorrichtungen mit Hornstrahleranordnung oder einer ECR-Plasmastromquelle sei beispielsweise auf die deutsche Patentanmeldung (das deutsche Patent) P 38 43 098.3 verwiesen.

Nachstehend wird die Erfindung durch ein Beispiel näher erläutert.

### Beispiel

Es wurden drei Skalpelle mit Hilfe der in der deutschen Patentanmeldung (dem deutschen Patent) P 37 38 352.3 beschriebenen Vorrichtung sterilisiert. Dazu wurde Sauerstoff als Medium gewählt und bei einem Druck von 10⁻¹ mbar gearbeitet. Die Einwirkungsdauer des Niederdruckplasmas betrug fünf min.

Unter dem Elektronenmikroskop ließen sich keine Spuren von Bakterien ermitteln.

## Patentansprüche

1. Verfahren zum Sterilisieren und Reinigen von Gegenständen von mikrobiologischen Verunreinigungen mit Hilfe eines Niederdruckplasmas, bei dem man das Niederdruckplasma durch eine eletromagnetische Strahlung von etwa 2,45 GHz in einem aldehydfreien Medium erzeugt, dadurch ***gekennzeichnet,*** daß man die eingesetzten Gegenstände bei der Sterilisation und Reinigung mit Hilfe eines IR-Strahlers bei erhöhter Temperatur hält und bei einem Druck im Bereich von 5 x 10¹ bis 0,5 x 10⁻⁴ mbar sterilisiert und reinigt.

2. Verfahren nach Anspruch 1, dadurch ***gekennzeichnet**,* daß man in einem Plasma von Sauerstoff, Stickstoff, Tetrafluormethan, Helium, Neon, Argon, Krypton oder einem Gemisch dieser Gase sterilisiert bzw. reinigt.

3. Verfahren nach einem der vorhergehenden Ansprüche, dadurch ***gekennzeichnet,*** daß man Gegenstände, wie Werkzeuge, Behälter oder deren Teile, die in der Medizin, der Biologie, der Lebensmitteltechnologie und/oder der Pharmazie Verwendung finden, sterilisiert und reinigt.

## Claims

1. A process for the sterilization of and cleaning of microbiological contaminants from articles using a low-pressure plasma, in which the low-pressure plasma is generated by elecromagnetic radiation of about 2.45 GHz in an aldehyde-free medium, characterised in that during the sterilisation and cleaning process the articles in question are maintained at elevated temperature using an IR radiator, and are sterilised and cleaned under a pressure in the range from 5 x 10¹ to 0.5 x 10⁻⁴ mbar.

2. A process according to claim 1, characterized in that the sterilization and cleaning are carried out in a plasma of oxygen, nitrogen, tetrafluoromethane, helium, neon, argon, krypton or a mixture of those gases.

3. A process according to any one of the preceding claims, characterized in that articles such as implements, containers or parts thereof that are used in medicine, biology, food technology and/or pharmacy are sterilized and cleaned.

## Revendications

1. Procédé permettant de stériliser et purifier des objets de leurs impuretés microbiologiques au moyen d'un plasma à basse pression, selon lequel on produit le plasma à basse pression au moyen d'un rayonnement électromagnétique de 2,45 GHz environ, dans un milieu dépourvu d'aldéhyde, caractérisé en ce que, lors de la stérilisation et de la purification, on maintient les objets, soumis au procédé, à une température assez élevée au moyen d'un dispositif de rayonnement infrarouge et en ce qu'on procède à la stérilisation et à la purification à une pression comprise entre 5 x 10¹ et 0,5 x 10⁻⁴ mbars.

2. Procédé selon la revendication 1, caractérisé en ce qu'on procède à la stérilisation et à la purification dans un plasma d'oxygène, d'azote, de tétrafluorométhane, d'hélium, de néon, d'argon, de crypton ou d'un mélange de ces gaz.

3. Procédé seon l'une des revendications précédentes, caractérisé en ce qu'on procède à la stérilisation et à la purification d'objets, tels qu'instruments, récipients ou parties de ces derniers, qui trouvent une application en médecine, en biologie, dans le traitement des produits alimentaires et/ou en pharmacie.
